# EUROPEAN PATENT APPLICATION

(11) **EP 3 384 827 A1**
(43) Date of publication of application: **10.10.2018**
(21) Application number: 17164577.3
(22) Date of filing: 03.04.2017
(51) Int. Cl.: A61B 5/00, A61B 5/024, A61B 5/11

(54) **SYSTEM FOR DETERMINING A PHYSIOLOGICAL PARAMETER OF A SUBJECT**

(71) Applicant: Koninklijke Philips N.V., 5656 AE Eindhoven (NL)
(72) Inventor: AARTS, Ronaldus Maria, 5656 AE Eindhoven (NL); KIRENKO, Ihor Olehovych, 5656 AE Eindhoven (NL); SHAN, Caifeng, 5656 AE Eindhoven (NL)
(74) Representative: de Haan, Poul Erik

(57) **Abstract**

The present invention relates to a system (1) for determining a physiological parameter of a subject (100). The system comprises a remote unit (11) comprising a sensor (12) for acquiring remote sensor data indicative of a motion and/or a physiological parameter of the subject; a wearable device (13) comprising a sensor (14) for acquiring wearable sensor data indicative of a physiological parameter of the subject; a control unit (15) configured to control acquisition of said wearable sensor data based on said remote sensor data; and a processing unit (16) configured to determine a physiological parameter of the subject based on at least one of said remote sensor data and said wearable sensor data. According to further aspects, a corresponding method, device and computer program are presented.

## Description

### FIELD OF THE INVENTION

The present invention relates to the field of medical technology and, in particular, to a system for determining a physiological parameter of a subject. The present invention further relates to a device for use in such a system, a corresponding method as well as a computer program for carrying out said method.

### BACKGROUND OF THE INVENTION

Vital signs of a person, for example the heart rate (HR), the respiration rate (RR) or the arterial blood oxygen saturation (SpO2), serve as indicators of the current health state of a person and as powerful predictors for serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave travelling through the body of a subject with every heartbeat. Photoplethysmography (PPG) is an optical measurement technique that evaluates a time-varying change of light reflectance or transmission of an area or volume of interest. PPG is based in the principle that blood absorbs light more than surrounding tissue, so that variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation (SpO2) can be determined.

One option for continuously monitoring a subject is the use of wearable biosensors. Wearable biosensors may offer low-cost, pervasive and long-term monitoring. However, a drawback of wearable biosensors is the limited battery life. A further drawback of biophysical sensors is their susceptibility for movement artifacts. A desired physiological signal can get seriously disturbed due to movement of the subject. This is in particular the case if the sensor evaluates a movement, such as an accelerometer-based respiration measurement.

Recently, non-contact, remote photoplethysmography (rPPG) devices (also called camera PPG devices) for unobtrusive measurement have been described in many publications, e.g. in Verkruysse et al., "Remote plethysmographic imaging using ambient light", Optics Express, 16(26), 22 December 2008, pp. 21434-21445, which demonstrates that photoplethysmographic signals can be measured remotely using ambient light and a conventional consumer level video camera, using red, green and blue color channels.

Remote PPG utilizes light sources or, in general radiation sources, disposed remotely from the subject of interest. Similarly, a detector, e.g., a camera or a photodetector, can be disposed remotely from the subject of interest, i.e., without contact to the subject as a non-contact, remote sensor. Therefore, remote photoplethysmographic systems and devices are considered unobtrusive and well-suited for medical as well as non-medical everyday applications.

While unobtrusive measurement modalities greatly increase comfort and number of application scenarios, the signal quality of a remote measurement depends severely on the measurement conditions and is subject to significant environmental disturbance.

In order to improve coverage and accuracy of an obtrusively measured biosignals, Antink et al. "Beat-to-beat heart rate estimation fusing multimodal video and sensor data", Biomedical Optics Express 2895, July 2015 teaches to fuse a remote PPG signal with a ballistocardiographic signal acquired with a mat placed on the seat of a chair that the patient has to sit on.

A disadvantage of this approach is limited flexibility since the subject has to remain in a well-defined position.

### SUMMARY OF THE INVENTION

It is an object of the present invention to address one or more of the aforementioned drawbacks. It would be advantageous to provide an improved system and method for determining a physiological parameter of a subject, in particular providing improved robustness regarding movement artifacts, improved monitoring accuracy and/or reduced power consumption.

In a first aspect of the present invention a system for determining a physiological parameter of a subject is presented. The system comprises:
- a remote unit comprising a sensor for acquiring remote sensor data indicative of a motion and/or a physiological parameter of the subject;
- a wearable device comprising a sensor for acquiring wearable sensor data indicative of a physiological parameter of the subject;
- a control unit configured to control acquisition of said wearable sensor data based on said remote sensor data; and
- a processing unit configured to determine a physiological parameter of the subject based on at least one of said remote sensor data and said wearable sensor data.

In a further aspect of the present invention a method for determining a physiological parameter of the subject is presented. The method comprises the steps of:
- acquiring remote sensor data indicative of a motion and/or a physiological parameter of the subject;
- acquiring wearable sensor data indicative of a physiological parameter of the subject;
- controlling acquisition of said wearable sensor data based on said remote sensor data; and
- determining a physiological parameter of the subject based on at least one of said remote sensor data and said wearable sensor data.

In a further aspect of the present invention a device for use in a system for determining a physiological parameter of a subject is presented. The device comprises: an input for obtaining remote sensor data indicative of a motion and/or a physiological parameter of the subject; an input for obtaining wearable sensor data indicative of a physiological parameter of the subject; and a control unit configured to control acquisition of said wearable sensor data based on said remote sensor data.

In yet further aspects of the present invention there are provided a corresponding a computer program which comprises program code means for causing a computer to perform the steps of the method disclosed herein when said computer program is carried out on a computer, as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, method, computer program and medium can have similar and/or identical preferred embodiments as the claimed system, in particular as defined in the dependent claims and as disclosed herein.

The present invention is based on the idea of making use of remote, non-contact monitoring for controlling acquisition of wearable sensor data using a wearable device. The inventors have recognized that measurement with a remote unit, such as a remote PPG camera, can in many cases provide reliable measurements. The proposed system thus comprises a remote unit comprising a sensor for acquiring remote sensor data indicative of a motion and/or a physiological parameter of the subject. Nonetheless, external circumstances, such as for example illumination conditions or the subject temporarily not being in a measurement range or field of view of the remote unit, limit measurement capabilities thereof. Such (temporal) gaps in acquiring measurement data can be filled by using a wearable device comprising a sensor for acquiring wearable sensor data indicative of a physiological parameter of the subject. However, a drawback of such wearable devices is power consumption and limited battery life. For example for a wearable device comprising a PPG sensor, the LEDs consume most of the power of the system. It is thus proposed to provide a control unit configured to control acquisition of said wearable sensor data based on said remote sensor data; and a processing unit configured to determine a physiological parameter of the subject based on at least one of said remote sensor data and said wearable sensor data. Hence, instead of fully parallel acquisition of data, the wearable device and in particular the sensor thereof can be controlled based on the remote sensor data. For example the control unit can control acquisition of said wearable sensor data by only activating the wearable device if needed.

A further concern in determining a physiological parameter of the subject is that sensors for acquiring data indicative of a physiological parameter can be susceptible to movement artifacts. The signal processing to determine whether a movement artifact may be present in the sensor data acquired by a wearable device may again consume a considerable amount of resources or would require additional sensing means to be employed on the wearable device. This would increase the complexity of the wearable device, may increase the power consumption and also increase the cost of the wearable device. Advantageously, such tasks may be outsourced such that it is not necessary to rely on sensor data of the wearable device. It is thus proposed to control acquisition of said wearable sensor data based on said remote sensor data instead.

Hence, a system is provided comprising a remote unit for acquiring remote sensor data indicative of a motion and/or a physiological parameter of the subject who is equipped with one or more wearable devices comprising a sensor for acquiring wearable sensor data indicative of a physiological parameter of the subject. However, the sensors do not operate independent of each other since a control unit is provided configured to control acquisition of said wearable sensor data based on said remote sensor data.

The term wearable device as used herein can refer to a device to by worn by the subject, in particular on the body of the subject, in particular attached to a skin of the subject. On the other hand, the term remote unit as used herein can refer to a non-contact, remote device that is not in direct contact with the subject. The remote unit can refer to a device for monitoring the subject from a distance.

In an embodiment the remote unit can comprise at least one camera. The camera comprises an image sensor configured to acquire a sequence of images as remote sensor data indicative of a motion and/or physiological parameter of the subject. For example, the camera can be used for remote PPG measurement, SpO2 measurement, and/or a respiratory motion of the subject by evaluating a respiratory motion. In addition or in the alternative, a motion of the subject can be determined as an indicator of motion artifacts to be expected in the signal of the wearable device.

In an embodiment, the sensor of the wearable device comprises at least one of a photoplethysmographic (PPG) sensor and a motion sensor. A wearable PPG sensor can enable contact-based PPG measurement and can thus provide a very reliable measurement. The sensor may be a transmission- or reflection-based PPG sensor. An exemplary motion sensor can refer to an accelerometer that may be either attached directly to a body part of the subject or may also be worn by attachment means or even in the clothing of the subject. Further sensor such as a temperature sensor (which may e.g. be disabled in response to a camera signal indicating that the subject moves towards a fireplace to avoid false measurements), galvanic skin response (GSR), electrocardiographic (ECG), or blood oxygen saturation (SpO2) sensor can be used. However, also others types of wearable biosensors or biophysical sensors can be used.

In an embodiment the control unit can be configured to extract a first physiological signal from said remote sensor data and to control acquisition of said wearable sensor data based on a quality of said first physiological signal. For example, if the quality of the first physiological signal based on said remote sensor data is good enough, the system may rely on the remote measurement. On the other hand, if the remote signal quality is not good enough, a measurement with the wearable device can be initiated. For example, if the subject goes out of a measurement range of the remote sensor (e.g. out of sight of a camera) or if this sensor does not provide a reliable signal, then the control unit can be configured to initiate acquisition of wearable sensor data indicative of the physiological parameter of the subject and the physiological parameter can be determined based thereon. Exemplary scenarios include a limited viewing angle or illumination or a low signal-to-noise ratio.

In a refinement, the control unit can be further configured to switch the wearable device into a power-saving mode if the quality of said first physiological signal exceeds a predetermined first quality threshold; and/or to switch the wearable device into a measurement mode for acquisition of wearable sensor data if the quality of said first physiological signal is below a predetermined second quality threshold. An advantage of this embodiment is that the wearable device is only used as needed such that the power consumption can be reduced significantly. The first and second threshold can be the same or different thresholds.

In the addition or in the alternative, the control unit can be configured to control a measurement accuracy of the wearable device such that a quality of a second physiological signal extracted from a combination of said remote sensor data and said wearable sensor data at least reaches a predetermined third quality threshold. This embodiment is based on the idea that if the remote sensor alone does not provide sufficiently good signal quality, the remote sensor data may be complemented with wearable sensor data. In particular, acquisition of said wearable sensor data can be controlled based on said remote sensor data such that the sensor data from both sensors together, i.e., the quality of the second physiological signal extracted from the combination, provides a sufficiently good signal quality. However, it is not mandatory that the wearable sensor data alone would be sufficiently good to use without further support of the remote sensor data. This is based on the idea that a highly accurate measurement usually requires more power, either for communicating of precise measurements results or for accurate operation of the sensor. For example, in case of a wearable PPG sensor, it may not be necessary to operate the light sources of the PPG sensor at full power but only to operate them at a sufficient power level to provide wearable sensor data that can supplement the remote sensor data, such that the combination enables reliable determination of the physiological parameter of the subject. Hence, in case of a bad signal-to-noise ratio, the data or signals of both sensors can be fused in order to get a reliable signal. Nonetheless, it is not necessary to provide stand-alone high accuracy signals such that the (battery-powered) wearable device - in which power consumption is an issue - can be operated at lower power consumption and in an embodiment only supply the missing signal quality portion.

In an embodiment the wearable device comprises a contact-based PPG sensor and a power of a light source of said PPG sensor can be controlled based on the quality of the first physiological signal. The first physiological signal can again be extracted from said remote sensor data. The control unit can be configured to control a power of the light source of the PPG sensor. An advantage of this embodiment is reduced power consumption. A quality of PPG measurement data scales with the signal-to-noise ratio (SNR). The SNR may be improved by increasing optical power at the expense of increased power consumption. In particular if there is already some information about the physiological parameter to be determined available based on the remote sensor data, additional wearable sensor data (at reduced power of the light source and thus lower power consumption of the wearable sensor) may be acquired to supplement said remote sensor data.

In an embodiment the sensor of the wearable device and the sensor of the remote unit can be configured to acquire wearable sensor data and remote sensor data indicative of the same physiological parameter. The processing unit can be configured to sequentially merge wearable sensor data and remote sensor data (in time) such that substantially continuous monitoring of the subject is provided. An advantage of this embodiment is that continuous monitoring of the subject can be ensured in that overall information can be gathered for the whole time frame. For example, a remote PPG camera monitors the subject as long as visual contact to a bare skin region is available. However, if the subject is not exposed to the camera, the control unit may activate the wearable sensor such that those temporal portions wherein the remote sensor is not capable of acquiring data are filled by data acquired by the wearable device. It will be appreciated that if the remote unit acquires remote sensor data, the wearable device can be switched into a power-saving mode. Hence, the remote senor data and the wearable sensor data may complement one another sequentially in time. The wearable sensor and the remote sensor may operate based on the same measurement principle (e.g. photoplethysmographic measurements) or different measurement principles (e.g. remote PPG and contact-based ECG) to acquire wearable sensor data and remote sensor data indicative of the same physiological parameter.

In an embodiment the processing unit can be configured to determine a physiological parameter of the subject based on a combination of said remote sensor data and said wearable sensor data. An advantage of this embodiment is further improved accuracy. Hence, instead of fully relying on one signal source, the combination of both the remote sensor data as well as the wearable sensor data may be used to improve one or more of accuracy, reliability and a signal-to-noise ratio. Optionally, the remote sensor data and the wearable sensor data can be fused based on weights indicative of the signal quality.

In an embodiment control unit it is configured to extract a motion signal indicative of a motion of the subject from said remote sensor data and to control acquisition of said wearable sensor data based on said motion signal. Advantages of this approach can include a reduction of false alarms and/or reduced power consumption of the wearable device. The motion signal can for example be indicative of an amount of motion of the subject to be monitored. The motion of the subject correlates with motion artifacts that can disturb the sensor data indicative of a physiological parameter of the subject. For the case that no motion of the subject or only a motion below a predetermined threshold is detected, the wearable sensor data can be considered to be reliable. On the other hand, if motion above a predetermined threshold is detected, this can be indicative of a reduced reliability or increased probability of motion artifacts. This information can be used to indicate that an unusual measurement value of the physiological parameter of the subject might be a false alarm and preferably be indicated to a clinician. For example, a different alarm signal may be presented to the clinician as compared to a situation where such an event is detected during a period of high signal acquisition quality. More generally speaking, an amount of motion of the subject can be estimated based on the remote sensor data and a functionality and/or output protocol of the wearable device can be adjusted based thereon. Depending on how critical the state of the subject to be monitored is, it also possible to only determine the physiological parameter of the subject in the absence of motion-based disturbances.

In a refinement, the control unit can be further configured to switch the wearable device into a power-saving mode if the motion signal exceeds a predetermined first motion threshold; and/or to switch the wearable device to a measurement mode for acquisition of wearable sensor data if the motion sensor is below a predetermined second motion threshold. An advantage of this embodiment is reduced power consumption. In other words, if the wearable device cannot be expected to produce reliable measurement data anyway, it can be switched into a power-saving mode to reduce power consumption. The first and the second motion thresholds can be the same or different thresholds. For example, a hysteresis can be implemented to avoid repeatedly toggling the wearable device between power-saving and measurement mode.

In an embodiment the sensor of the wearable device can be a sensor for acquiring wearable sensor data indicative of a respiration or pulse-rate of the subject.

The herein presented solution does provide a possibility to preferably achieve one or more of an improved robustness, improve accuracy of monitoring and/or reduced power consumption of a wearable device.

### BRIEF DESCRIPTION OF THE DRAWINGS

These and other aspects of the invention will be apparent from and elucidated with reference to the embodiment(s) described hereinafter. In the following drawings
Fig. 1 schematically shows a first embodiment of a system according to an aspect of the present invention,
Fig. 2 shows a block diagram of a system and device according to an aspect of the present invention;
Fig. 3 shows an exemplary embodiment of a wearable device;
Fig. 4 shows a flow chart of a method according to an aspect of the present invention;
Fig. 5 shows a flow chart of a first embodiment of a process of interaction of the remote unit and the wearable device;
Fig. 6 shows a first embodiment of exemplary signals based on remote sensor data and wearable sensor data;
Fig. 7 shows a flow chart of a second embodiment of a process of interaction of the remote unit and the wearable device; and
Fig. 8 shows a second embodiment of exemplary signals based on remote sensor data and wearable sensor data.

### DETAILED DESCRIPTION OF THE INVENTION

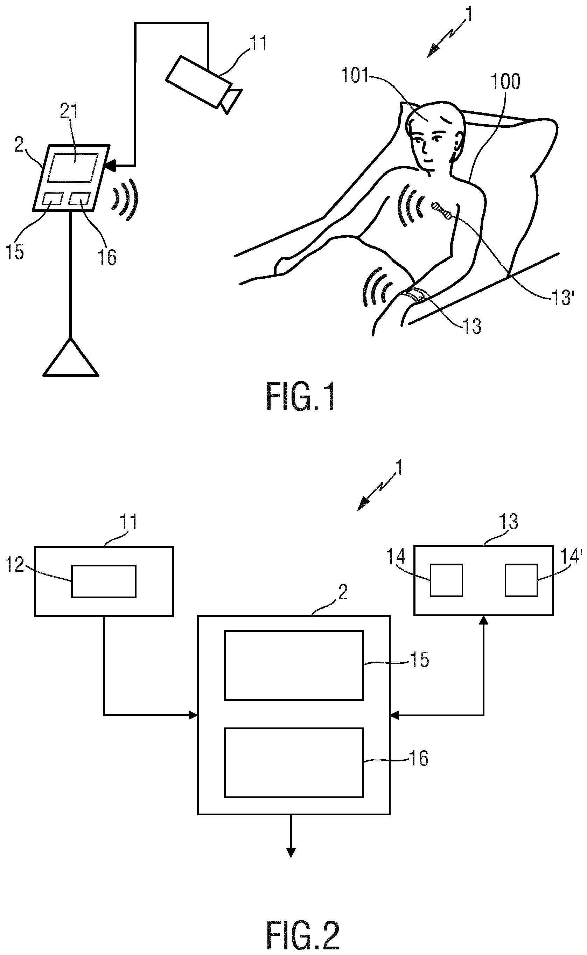

Fig. 1 schematically shows a first embodiment of a system according to an aspect of the present invention. The system is therein denoted in its entirety by reference numeral 1. Fig. 2 shows a block diagram which schematically illustrates the components of the system 1 according to the first embodiment shown in Fig. 1. The system 1 for determining a physiological parameter of subject 100 comprises a remote unit 11 comprising a sensor 12 for acquiring remote sensor data indicative of a motion and/or a physiological parameter of the subject; a wearable device 13 comprising a sensor 14 for acquiring wearable sensor data indicative of a physiological parameter of the subject; a control unit 15 configured to control acquisition of said wearable sensor data based on said remote sensor data; and a processing unit 16 configured to determine a physiological parameter of the subject based on at least one of said remote sensor data and said wearable sensor data.

In the embodiment shown in Fig. 1, the remote unit 11 is a camera comprising an image sensor 12 for acquiring remote sensor data. The remote sensor data can be a series of images. In a first application scenario, the image data can be processed using known techniques in the field of remote photoplethysmography (remote PPG), as for example described by Verkruysse et al., in "Remote plethysmographic imaging using ambient light", Optics Express, 16(26), 22 December 2008, pp. 21434-21445. For example, a pulse rate and/or blood oxygen saturation of the subject can be determined as the physiological parameter. In a second application scenario, the image data can be processed to evaluate an amount of motion of this subject. The remote unit can also comprise a plurality of sensors, e.g. a plurality of cameras, for improved coverage.

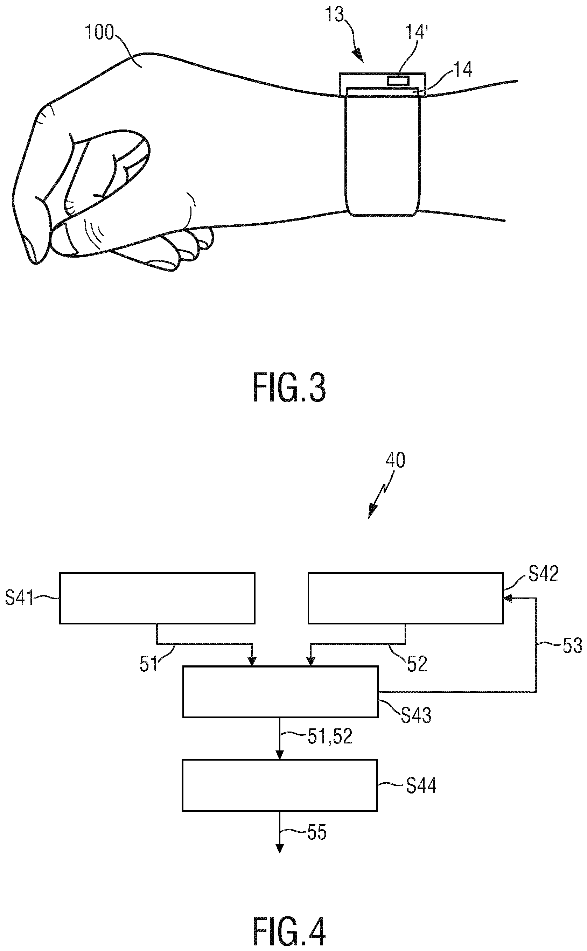

Fig. 1 further shows a first and a second wearable device 13, 13' attached to the body of the subject. The first wearable device 13 is implemented in form of a wrist-mounted wearable device as shown in more detail in Fig. 3. In the shown embodiment, the wearable device 13 comprises a contact-based PPG sensor 14. The PPG sensor acquires sensor data indicative of a cardiac activity, in particular the pulse (rate), of the subject 100. Optionally, the contact-based PPG sensor 14 may be configured to acquire sensor data indicative of the blood oxygen saturation (SpO2).

In addition or in the alternative, the wearable device may comprise an accelerometer 14' configured to acquired wearable sensor data based on a motion of the subject.

The second wearable device 13' is implemented in form of a patch to be applied to the body of the subject 100. In the shown embodiment, the patch is applied to a chest of the subject. The wearable device 13' can comprise an accelerometer 14' configured to acquire wearable sensor data based on a motion of the subject. In the shown embodiment, a chest motion of the subject 100 indicative of a respiratory movement can be evaluated to determine a respiration rate of the subject. Other types of sensors, such as an ECG sensor may be implemented in addition or in the alternative.

In the embodiment shown in Fig. 1 and Fig. 2, the control unit 15 and optionally also the processing unit 16 form part of a device 2 for use in the system 1 for determining a physiological parameter of a subject 100. The device 2 is configured to communication wired or wirelessly with the remote unit 11 and the wearable devices 13, 13'. The device 2 can thus comprise a first input for obtaining (i.e. receiving or retrieving) remote sensor data indicative of a motion and/or a physiological parameter of the subject from the remote unit 11; and a second input for obtaining wearable sensor data indicative of a physiological parameter of the subject from at least one of the wearable devices 13, 13'. The device 2 may be provided as a stand-alone device or may be co-integrated with other devices such as e.g. a patient monitor or other (medical) device, such as e.g. the remote unit 11. The control unit 15 and the processing unit 16 can be understood as functional units, that can be implemented separately or may also be co-integrated for example embodied in form of a microcontroller configured to perform the respective functionality or even as a server or cloud-based embodiment.

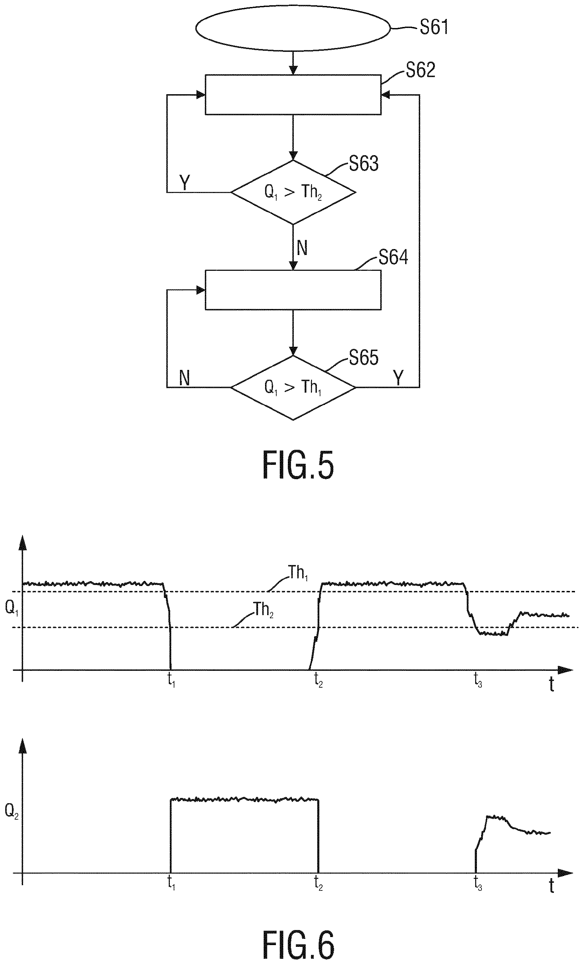

The control unit 15 configured to control acquisition of the wearable sensor data with the wearable device 13 based on the remote sensor data acquired by the remote unit 11. For example, the control unit may switch the wearable device 13 into a power-saving mode if the quality of the remote data is good enough to reliably determine the physiological parameter. Hence, the power consumption of the wearable device can be reduced and the battery life of the wearable device extended. Two more detailed scenarios will be explained in more detail further below with reference to Fig. 5 to 8.

Fig. 4 shows a flow chart of a method 40 for determining a physiological parameter of a subject according to an aspect of the present invention. The method comprises the steps of obtaining remote sensor data 51 indicative of a motion and/or a physiological parameter of the subject (S41); obtaining wearable sensor data 52 indicative of a physiological parameter of the subject (S42); controlling acquisition of said wearable sensor data based on said remote sensor data (S43); and determining a physiological parameter 55 of the subject based on at least one of said remote sensor data 51 and said wearable sensor data 52 (S44). A control signal 53 can be provided to control the acquisition of the wearable sensor data 52 based on the remote sensor data 51.

In step S43, the remote sensor data 51 is provided as an input for controlling the wearable device based thereon. Exemplary scenarios will be described below with reference to Fig. 5-8. Optionally, also the wearable sensor data 52 can be used as a further input in the step S43, for example for establishing a feedback loop for controlling the acquisition of the remote sensor data such that a quality of a physiological signal extracted from a combination of said remote sensor data and said wearable sensor data at least reaches a predetermined third quality threshold.

In step S44, a physiological parameter of the subject is determined based on at least one of said remote sensor data 51 and said wearable sensor data 52. Advantageously the remote sensor data 51 and the wearable sensor data 52 can be merged sequentially (merged in time) such that periods, where there is no reliable remote sensor data 51 available, are complemented by wearable sensor data 52.

A first application scenario is described with reference to Fig. 5 and Fig. 6. In this first application scenario the remote unit 11 comprises a camera for remote PPG and/or SpO2 measurement as well as a wearable device for contact-based PPG and/or SpO2 measurement. The measurement process starts in Fig. 5 at step S61. In process step S62, the remote unit 11 acquires remote sensor data with the camera and the control unit 15 extracts a first physiological signal, here a heart rate signal and optionally a blood oxygen saturation signal, from the remote sensor data. A quality of said first physiological signal is indicated on the vertical axis in the upper graph in Fig. 6 and denoted by Q₁. The horizontal axis denotes the time t.

If the signal measured by the remote unit is a reliable signal, the wearable device can be switched into a power-saving mode. For example, a light source in the wearable device sensors may be switched off to reduce power consumption.

In decision step S63, the quality of said first physiological signal is compared with a threshold Th2. If the quality of the signal measured by the remote unit exceeds the threshold Th2, i.e., fulfills the condition Q1>Th2, then the remote signal acquisition already provides a reliable signal. Hence, the wearable device may either not be activated after all or may be switched into a power-saving mode. The process may thus loop back to step S62 and proceed with the remote measurement.

However, if the quality of the first physiological signal is below the threshold, i.e., no longer fulfills the condition Q1>Th2. Then the wearable device is switched into a measurement mode for acquisition of wearable sensor data, as indicated by S64. This condition applies at time t₁ in Fig. 6. For example, as soon as the patient gets out of the sight of the camera, or the camera monitoring cannot derive a reliable PPG or SpO2 signal, the control unit can initiate acquisition of wearable sensor data by the wearable device. For camera-based PPG monitoring, this can be due to bad view angle or illumination for measurement, or less skin visible in the camera field of view. For camera-based respiration monitoring, this happens when the belly/chest breathing movements are not visible in the camera view, for instance, due to view angle.

As indicated in the lower graph in Fig. 6, the sensor of the wearable device may provide a very good signal quality, indicated by the vertical axis Q2.

Once the signal quality of the first physiological signal, acquired by the remote unit 11, has recovered, the wearable device may again be switched into a power-saving mode at time t₂. Hence, if the quality of the signal measured by the remote unit exceeds the threshold Th1, i.e., fulfills the condition Q1>Th1 in step S65, then the remote signal acquisition already provides a reliable signal and the process may resume back to step S62. The thresholds Th1 and Th2 can be the same. However, advantageously different thresholds can be used avoid repeatedly toggling the wearable device between power-saving and measurement mode.

In an optional refinement, the control unit 15 can be configured to control acquisition of the wearable sensor data in a more sophisticated way. As indicated in Fig. 6, if the signal quality Q1 of the first physiological signal extracted purely based on the remote sensor data drops below a threshold Th1, the control unit can active and control the wearable device to provide supplementary wearable sensor data to complement the remote sensor data (starting at time t₃ in Fig. 6). For example, the control unit can be configured to control a measurement accuracy of the wearable device such that a quality of a second physiological signal extracted from a combination of said remote sensor data and said wearable sensor data at least reaches a predetermined third quality threshold. For example, in case of a bad signal to noise ratio (SNR), both the remote and the wearable sensors can be fused in order to get a reliable signal. In a simple case a weighted average can be used, where the weights are determined by the signal to noise ratios or a surrogate measure of it of the individual signals. For example, in case of a wearable PPG sensor, a power of the light source may be reduced since only a complementary wearable sensor data is required to complement the remote sensor data for determining the physiological parameter of the subject.

As can be seen from the signal traces in the upper and lower graph in Fig. 6, the remote sensor data acquired by the remote unit 11 and the wearable sensor data acquired, controlled based on the remote sensor data, by the wearable device 13, complement each other over time, such that the wearable sensor data and remote sensor data can be sequentially merged by the processing unit 16 such that substantially continuous monitoring of the subject is provided. The processing unit is configured to determine the physiological parameter of the subject based on sensor data indicative of said physiological parameter acquired by at least one of said remote sensor or said wearable sensor.

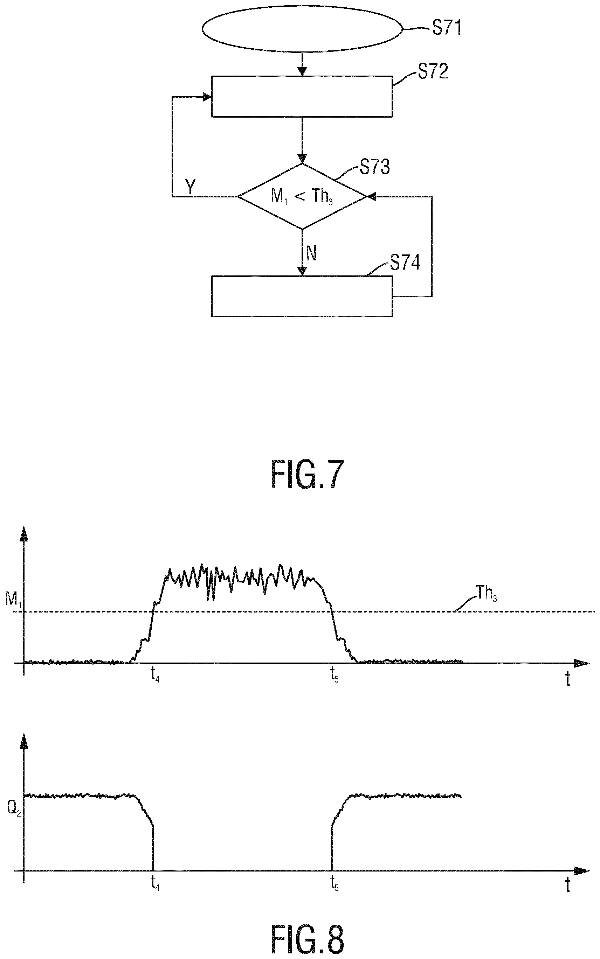

A second application scenario is described with reference to Fig. 7 and Fig. 8. In this application scenario, the remote unit 11, for example comprising a camera, estimates the amount of motion of a body of the subject and the control unit 15 adjusts the functionality and the output protocol of the wearable device based thereon. For instance, an accelerometer-based sensor, or an ECG patch provide a reliable estimation of a respiratory effort only in the absence of non-respiratory body motions. Distinction between respiratory and non-respiratory motions can be achieved by means of analysis of the strength of the acquired remote data indicative of a motion of the subject. However, this approach might lead to interpretation of a deep breath as a body motion, and small body motion as a shallow breathing. Therefore, the additional embodiment of the disclosure proposes to detect and estimate the body motion using the remote (camera-based) unit and control acquisition by the wearable device accordingly. For instance, if a camera system detects motion of a patient' body, it can send a control signal to a wearable device to switch off, thus saving an energy of the wearable device and avoid false alarms.

Referring again to Fig 7 and Fig. 8, the measurement process starts in Fig. 7 at step S71. The wearable device comprises a sensor for acquiring wearable sensor data indicative of a physiological parameter of the subject, here a respiration sensor, in step S72. In parallel, the remote unit acquires sensor data indicative of a motion of the subjection. The control unit extracts a motion signal based on the remote sensor data, as indicated in the upper graph in Fig. 8. The horizontal axis denotes time t, whereas the vertical axis denotes a motion magnitude. The lower graph in Fig. 8 denotes the signal quality Q2 of a physiological signal extracted based on the remote sensor data.

In step S73, the control unit compares the motion signal M1 with a predetermined motion threshold Th3. The control unit is configured to switch the wearable device into a power-saving mode if the motion signal exceeds the predetermined motion threshold (see time t₄); and/or to switch the wearable device into to a measurement mode for acquisition of wearable sensor data if the motion signal is below the predetermined motion threshold Th3 (see time t₅). Alternatively, different motion thresholds may be used similar to threshold comparison described with reference to the first application scenario. If the motion signal is above the predetermined motion threshold, the process may enter an idle state S74 and loop back to the threshold comparison in step S73.

In conclusion, the present disclosure provides an improved system and method for determining a physiological parameter of a subject, in particular providing improved robustness regarding movement artifacts, improved monitoring accuracy and reduced power consumption.

The solutions presented herein can find advantageous applications in neonate intensive care units (NICU) continuous monitoring or spot-check of newborn babies, monitoring in low-acuity settings, and further applications, such as tele-monitoring at home by means of a tablet with an embedded camera.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. System (1) for determining a physiological parameter of a subject (100), the system comprising:
- a remote unit (11) comprising a sensor (12) for acquiring remote sensor data indicative of a motion and/or a physiological parameter of the subject;
- a wearable device (13) comprising a sensor (14) for acquiring wearable sensor data indicative of a physiological parameter of the subject;
- a control unit (15) configured to control acquisition of said wearable sensor data based on said remote sensor data; and
- a processing unit (16) configured to determine a physiological parameter of the subject based on at least one of said remote sensor data and said wearable sensor data.

2. System as claimed in claim 1, wherein the remote unit (11) comprises at least one camera.

3. System as claimed in claim 1, wherein the sensor (14) of the wearable device (13) comprises at least one of a photoplethysmographic (PPG) sensor and a motion sensor.

4. System as claimed in claim 1, wherein the control unit (15) is configured to extract a first physiological signal from said remote sensor data and to control acquisition of said wearable sensor data based on a quality of said first physiological signal.

5. System as claimed in claim 4, wherein the control unit (15) is further configured to switch the wearable device (13) into a power-saving mode if the quality of said first physiological signal exceeds a predetermined first quality threshold; and/or to switch the wearable device (13) into to a measurement mode for acquisition of wearable sensor data if the quality of said first physiological signal is below a predetermined second quality threshold.

6. System as claimed in claim 4, wherein the control unit (15) is configured to control a measurement accuracy of the wearable device (13) such that a quality of a second physiological signal extracted from a combination of said remote sensor data and said wearable sensor data at least reaches a predetermined third quality threshold.

7. System as claimed in claim 4, wherein the wearable device (13) comprises a contact-based PPG sensor and wherein a power of a light source of said PPG sensor is controlled based on the quality of the first physiological signal.

8. System as claimed in claim 1, wherein the sensor (14) of the wearable device (13) and the sensor (12) of the remote unit (11) are configured to acquire wearable sensor data and remote sensor data indicative of the same physiological parameter and wherein the processing unit (16) is further configured to sequentially merge wearable sensor data and remote sensor data in time such that substantially continuous monitoring of the subject is provided.

9. System as claimed in claim 1, wherein the processing unit (16) configured to determine a physiological parameter of the subject (100) based on a combination of said remote sensor data and said wearable sensor data.

10. System as claimed in claim 1, wherein the control unit (15) is configured to extract a motion signal indicative of a motion of the subject (100) from said remote sensor data and to control acquisition of said wearable sensor data based on said motion signal.

11. System as claimed in claim 10, wherein the control unit (15) is further configured to switch the wearable device (13) into a power-saving mode if the motion signal exceeds a predetermined first motion threshold; and/or to switch the wearable device (13) into to a measurement mode for acquisition of wearable sensor data if the motion signal is below a predetermined second motion threshold.

12. System as claimed in claim 10, wherein said sensor (14) of the wearable device (13) is a sensor for acquiring wearable sensor data indicative of a respiration or pulse rate of the subject (100).

13. Device (2) for in a system (1) for determining a physiological parameter of a subject (100), the device comprising:
- an input for obtaining remote sensor data indicative of a motion and/or a physiological parameter of the subject;
- an input for obtaining wearable sensor data indicative of a physiological parameter of the subject; and
- a control unit (15) configured to control acquisition of said wearable sensor data based on said remote sensor data.

14. A method (40) for determining a physiological parameter of a subject (100), the method comprising the steps of:
- obtaining remote sensor data indicative of a motion and/or a physiological parameter of the subject (S41);
- obtaining wearable sensor data indicative of a physiological parameter of the subject (S42);
- controlling acquisition of said wearable sensor data based on said remote sensor data (S43); and
- determining a physiological parameter of the subject based on at least one of said remote sensor data and said wearable sensor data (S44).

15. Computer program comprising program code means for causing a computer to carry out the steps of the method (40) as claimed in claim 14 when said computer program is carried out on the computer.
